# EUROPEAN PATENT SPECIFICATION

(11) **EP 3 797 825 B1**
(45) Date of publication and mention of the grant of the patent: **09.11.2022**
(21) Application number: 21152958.1
(22) Date of filing: 22.01.2021
(51) Int. Cl.: A61N 1/36, A61N 5/06

(54) **TEMPERATURE-CONTROLLED RADIO FREQUENCY COSMETIC DEVICE**
TEMPERATURGESTEUERTE KOSMETISCHE HOCHFREQUENZVORRICHTUNG
DISPOSITIF COSMÉTIQUE À RADIOFRÉQUENCE À TEMPÉRATURE CONTRÔLÉE

(30) Priority: 10.11.2020 CN 202022592883 U
(43) Date of publication of application: 31.03.2021
(73) Proprietor: Excelsior Medical Group, Kempston Bedford, Bedforshire MK42 7PW (GB)
(72) Inventor: Panesar, Bupinder, Bedford, Bedfordshire MK42 7PW (GB); Price, Richard, Bedford, Berkshire MK42 7PW (GB)
(74) Representative: Isern Patentes y Marcas S.L.

(56) References cited:
- CN-U- 203 971 193
- US-A1- 2010 010 480
- US-A1- 2013 066 237
- US-A1- 2015 230 863

## Description

### BACKGROUND OF INVENTION

The present disclosure particularly relates to a temperature-controlled radio frequency cosmetic device.

### Description of Related Art

Along with the improvement of living standards and the development of science and technology, the improved beauty caring and cosmetology are correspondingly required. Among them, the treatment that is heating the subcutaneous tissue of the human body with the temperature higher than the normal physiological body is popular, stimulating the human natural process of modifying or repairing the skin appearance with non-destructive and non-necrotizing temperature , and reducing subcutaneous fat and the like.

At present, it is common to heat the skin for treatment through radio frequency cosmetic technology that directly penetrates the skin and directly acts on subcutaneous tissue through RF energy, the radio frequency energy can enable cellular elements to generate strong resonant rotation to produce heat energy so as to achieve the purposes of heating collagen tissue and fat cells, resulting in instant temperature rise of the bottom layer of the skin, and the temperature rise stimulation of the dermis layer may cause instant collagen tightening and stimulate collagen regeneration so that the technology has the effects of improving skin tightening, wrinkle removing and face thinning.

However, in the existing radio frequency treatment and cosmetic processes, the temperature of the treated tissue cannot always be kept at the appropriate temperature, such that if the temperature is too low, the best treatment effect cannot be achieved, and if the temperature is too high, the skin and treated tissue could be damaged. CN203971193U discloses a lattice radio frequency cosmetic instrument which has a work control module that is connected with a control device which is arranged in main portion, and an information induction module which is comprised with induction unit and sensing head.

### SUMMARY OF INVENTION

The present disclosure has been put forward in view of the above-mentioned state of the prior art, and the object of the present disclosure is to provide a temperature-controlled radio frequency cosmetic device capable of monitoring the surface temperature of the treated tissue in real-time and keeping at appropriate temperature.

The invention is defined in the appended claims. Aspects, embodiments and examples disclosed herein which do not fall within the scope of the appended claims do not form part of the invention, and are merely provided for illustrative purposes. Furthermore, the methods presented in the present description are merely provided for illustrative purpose only and do not form part of the present invention.

According to the present disclosure, it is to provide a temperature-controlled radio frequency cosmetic device monitoring the temperature of a surface of a tissue to be treated in real-time and keeping at appropriate temperature.

### BRIEF DESCRIPTION OF THE DRAWINGS

Embodiments of the present disclosure will now be explained in further detail, by way of example only, with reference to the accompanying drawings, where:
Fig. 1 is a view showing an application scenario of a temperature-controlled radio frequency cosmetic device according to an embodiment of the present disclosure.
Fig. 2 is a schematic view showing a structure of a temperature-controlled radio frequency cosmetic device according to an embodiment of the present disclosure.
Fig. 3 is a distribution view showing a stimulation head according to an embodiment of the present disclosure.
Fig. 4 is a schematic block view showing a temperature-controlled radio frequency cosmetic device with a cover according to an embodiment of the present disclosure.
Fig. 5 is a schematic block view showing a temperature-controlled radio frequency cosmetic device according to an embodiment of the present disclosure.

### DETAILED DESCRIPTION

Hereinafter, the present disclosure will be described in further detail with reference to the accompanying drawings and specific embodiments. In the drawings, the same reference numerals are used for the same parts or parts having the same function, and a repeated description thereof will be omitted.

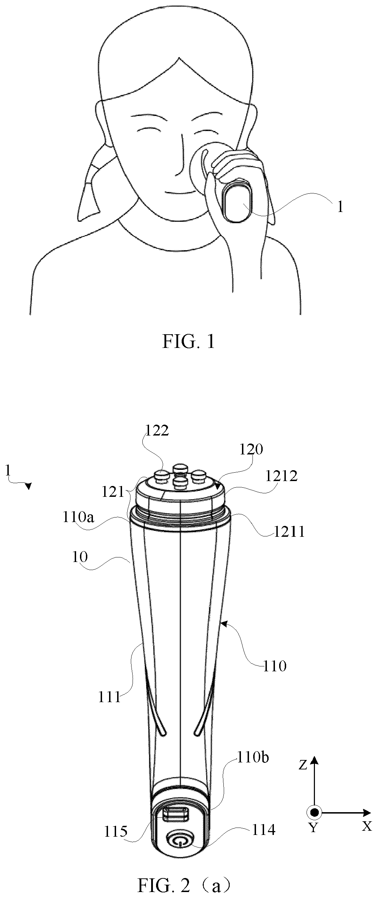

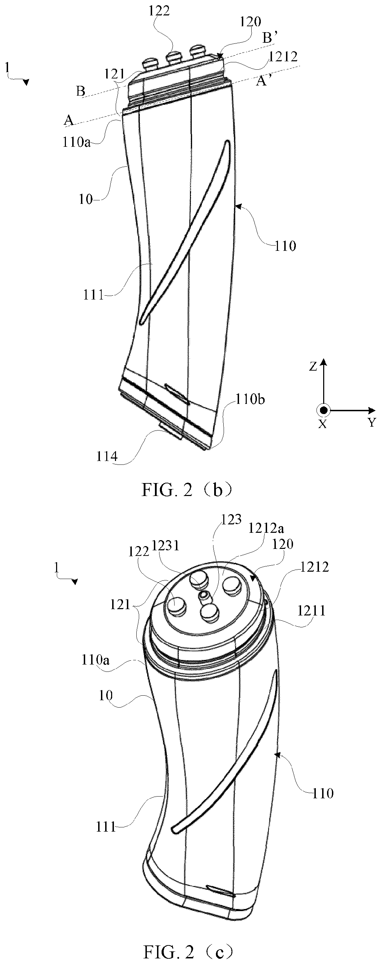

The present disclosure provides a temperature-controlled radio frequency cosmetic device 11 (see FIGS. 1 and 2). The temperature-controlled radio frequency cosmetic device 11 according to the embodiment of the present disclosure can apply radio frequency energy to the skin of a user so as to achieve the purpose of heating, resulting in cosmetic treatment on the skin. Since the temperature at which the skin is heated affects the overall treatment effect of the temperature-controlled radio frequency cosmetic device 11, in the present embodiment, the temperature-controlled radio frequency cosmetic device 11 can perform real-time temperature detection on the skin surface through a temperature sensing module 123 while applying radio frequency energy to the skin to ensure that the skin surface can be kept at appropriate temperature.

Hereinafter, the temperature-controlled radio frequency cosmetic device 1 according to the present disclosure will be described in detail with reference to the accompanying drawings.

Fig. 1 is a view showing an application scenario of a temperature-controlled radio frequency cosmetic device 1 according to an embodiment of the present disclosure.

In some examplary embodiments, the temperature-controlled radio frequency cosmetic device 1 may be handheld for use by an user (see Fig. 1). During the use of the temperature-controlled radio frequency cosmetic device 1, the temperature-controlled radio frequency cosmetic device 1 could be configured to apply radio frequency energy to the skin of the user. In particular, when the temperature-controlled radio frequency cosmetic device 1 is used, the user can handhold the temperature-controlled radio frequency cosmetic device 1 and enable the stimulation head 122 of the temperature-controlled radio frequency cosmetic device 1 and the probe end 1231 of the temperature sensing module 123 to contact the skin surface (such as a facial surface or the like) to be treated, and then the radio frequency energy can be applied to the skin to achieve the purpose of heating for the cosmetic skin treatment.

Fig. 2 is a schematic diagram showing a structure of a temperature-controlled radio frequency cosmetic device 1 according to an embodiment of the present disclosure. Wherein, Fig. 2 (a) is a perspective structural view of the temperature-controlled radio frequency cosmetic device 1. Fig. 2 (b) is a perspective structural view of the temperature-controlled radio frequency cosmetic device 1 of Fig. 2 (a) at a different viewing angle. In the embodiment shown in Fig. 2, a detailed description can be made based on an XYZ orthogonal coordinate system (see FIG. 2 (a)). The direction X may correspond to a horizontal left-right direction, the direction Y may correspond to a horizontal front-rear direction, and the direction Z may correspond to a vertical up-down direction. The direction Y and the direction Z are orthogonal to the direction X, respectively. Sometimes the direction X is labeled left or right, the direction Y is labeled front or rear, and the direction Z is labeled up or down.

In the embodiment according to the present disclosure, the shape of a main body portion 110 may not be so much limited , wherein one end of the main body portion 110 may be generally formed in a shape to facilitate holding, and the other end may be formed in a shape to carry the head 120. A related embodiment of the main body portion 110 will be described in detail with reference to the accompanying drawings.

In some examplary embodiments, the temperature-controlled radio frequency cosmetic device 1 may include a main body housing 10 (see FIGS. 1 and 2). In some examplary embodiments, the main body housing 10 may include a main body portion 110 and a head 120 (see Fig. 2). Wherein, the head 120 may be connected to the main body portion 110.

In some examplary embodiments, the main body portion 110 may include a first housing 111 having a first hollow structure. In some examplary embodiments, the first housing 111 may be formed substantially from a resin material such as PS757K ABS plastic using a molding process. In some examplary embodiments, the head 120 may include a second housing 121 having a second hollow structure. In some examplary embodiments, the second housing 121 may be made substantially of stainless steel material, such as 304 stainless steel.

In some examplary embodiments, the first housing 111 and the second housing 121 may be mechanically connected to each other. For example, the first housing 111 and the second housing 121 may be connected to each other through adhering and/or engaging. In some examplary embodiments, the first hollow structure and the second hollow structure may be communicated.

In some examplary embodiments, the first housing 111 may have a distal side 111b remote from the second housing 121. The first housing 111 may also have a proximal side 111a near the second housing 121.

In some examplary embodiments, the first housing 111 may be substantially columnar. In some examplary embodiments, the shape of the end face of the distal end side 111b of the first housing 111 may be substantially elliptical, and the shape of the end face of the proximal end side 111a of the first housing 111 may be substantially circular (see Fig. 2). In some examplary embodiments, the end face of the distal end side 111b of the first housing 111 may smoothly transit toward the end face of the proximal end side 111a to form the first housing 111. In this case, it is possible to both facilitate the user to handhold the temperature-controlled radio frequency cosmetic device 1, and to dispose the head 120 on the main body portion 110 more effectively.

In some examplary embodiments, the second housing 121 may be substantially columnar. In some examplary embodiments, the second housing 121 may have a connecting end 1211 connected to the first housing 111 and a functional end 1212 connected to the connecting end 1211. In some examplary embodiments, the connecting end 1211 may be substantially flat columnar. In some examplary embodiments, the cross-sectional shape of the connecting end 1211 in the direction of line AA' may match the shape of the end face of the proximal end side 111a. For example, the cross-sectional shape of the connecting end 1211 in the direction of line AA' may be the same as the end face of the proximal end side 111a. The cross-sectional shape of the connecting end 1211 on line AA' may be substantially circular.

In some examplary embodiments, the second housing 121 may be mechanically connected to the first housing 111. For example, in some examplary embodiments, the end face of the proximal end side 111a of the first housing 111 may have a window communicating with the first hollow structure, and the lower end face of the connecting end 1211 may be provided with a protrusion matching the window, in that case the connecting end 1211 can be connected with the first housing 111 in an engagement manner.

In some examplary embodiments, the head 120 may also have a functional end 1212, as described above. In some examplary embodiments, the functional end 1212 may be substantially columnar. In some examplary embodiments, the functional end 1212 may be disposed so as to protrude upward from an upper surface of the connecting end 1211 (see Fig. 2 (a)). In some examplary embodiments, the cross-sectional shape of functional end 1212 on line BB' may be substantially circular. In some examplary embodiments, the edge portion of the functional end 1212 may be a rounded transition.

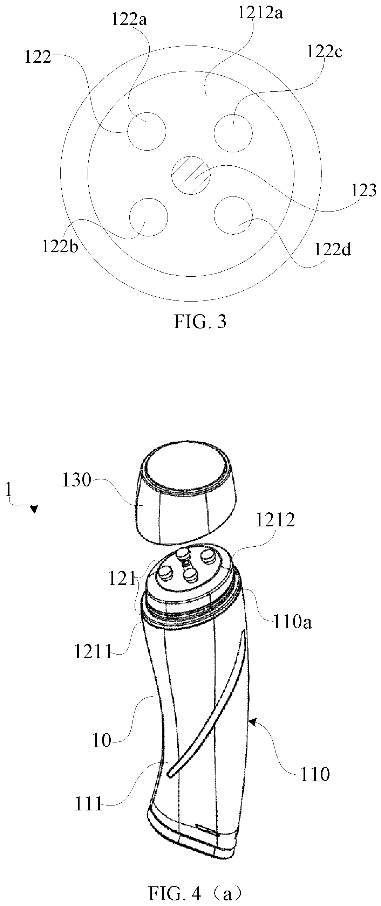

Fig. 3 is a schematic diagram showing a distribution of a stimulation head 122 according to an embodiment of the present disclosure.

In some examplary embodiments, the temperature-controlled radio frequency cosmetic device 1 may include a stimulation head 122 for applying radio frequency energy to the skin. Wherein, the stimulation head 122 may be configured on the head 120. In some examplary embodiments, the stimulation head 122 may be disposed on the upper surface 1212a of functional end 1212. For example, in some examplary embodiments, the stimulation head 122 may be disposed to protrude upward from the upper surface 1212a of functional end 1212. Alternatively, the stimulation head 122 may be disposed to be flush with the upper surface 1212a of the functional end 1212. In some examplary embodiments, the stimulation head 122 may be fabricated from metal or other conductive material. For example, the stimulation head 122 may be fabricated from 304 stainless steel. In some examplary embodiments, the number of stimulation heads 122 may be one or more. In some examplary embodiments, the protrusion height of the plurality of stimulation heads 122 relatives to the upper surface 1212a of the functional end 1212 may be the same. This enables the stimulation head 122 to stimulate the skin more effectively. For example, in some examplary embodiments, the stimulation head 122 may be a plurality of point electrodes (see FIGS. 2 and 3). In some examplary embodiments, the plurality of point electrodes may be uniformly distributed on the upper surface 1212a. For example, the plurality of point electrodes may be arranged on the upper surface 1212a in a regular row and column matrix. The plurality of point electrodes may also be arranged in concentric circles.

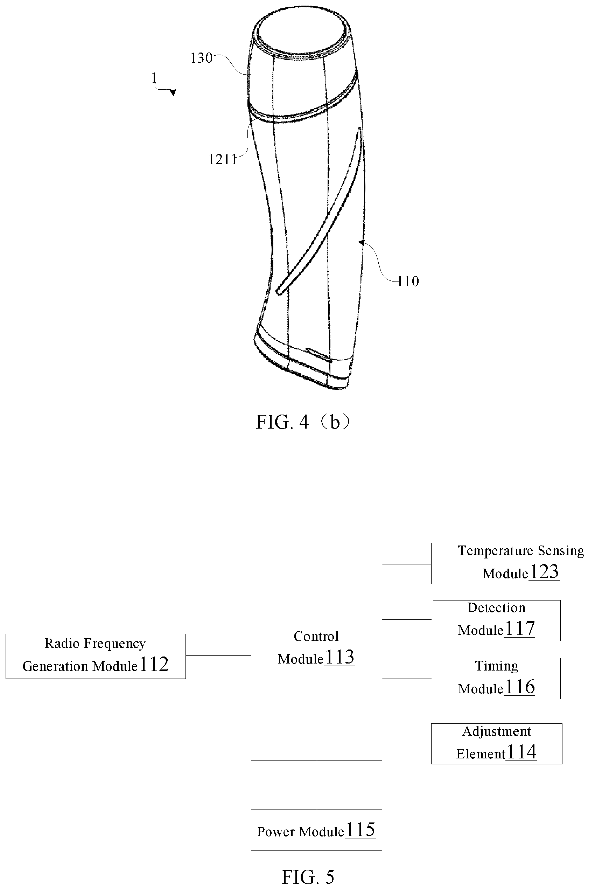

In some examplary embodiments, a radio frequency generation module 112 (see Fig. 5) may include a plurality of radio frequency output points, each radio frequency output point corresponds to the plurality of electrodes in the stimulation head. In this case, the skin can be heated more effectively. For example, in some examplary embodiments, the stimulation head 122 may include a plurality of point electrodes, and the plurality of point electrodes may be divided into a plurality of electrode groups. In some examplary embodiments, each electrode group may include at least two point electrodes. In some examplary embodiments, the point electrodes located in different electrode groups may share one radio frequency output point of the radio frequency generation module 112 (described in detail below). In some examplary embodiments, the polarity of the point electrodes sharing the same radio frequency output point may be the same. In some examplary embodiments, when the plurality of electrode groups are arranged on the upper surface 1212a, the point electrodes sharing the same radio frequency output point may be arranged at relatively remote locations, respectively. Therefore, the temperature-controlled radio frequency cosmetic device 1 can be prevented from heating the skin too fast, and the temperature-controlled radio frequency cosmetic device 1 can be used more safely. For example, referring to Fig. 3, the stimulation head 122 may include four point electrodes, which are divided into two electrode groups, wherein the point electrode 122a and point electrode 122b may be located in the same electrode group, the point electrode 122c and point electrode 122d may be located in the same electrode group, and the four point electrodes may be arranged on the upper surface 1212a in a rectangular distribution manner in which the radio frequency generation module 112 may include two radio frequency output points, the point electrode 122a may share one radio frequency output point with the point electrode 122d, the point electrode 122b may share another radio frequency output point with the point electrode 122c, the point electrode 122a and the point electrode 122d may be located at opposite corners of a rectangle, and the point electrode 122b and the point electrode 122c may be located at opposite corners of a rectangle.

In some examplary embodiments, when an electrode group receives radio frequency energy from the radio frequency generation module 112, at least two electrodes of opposite polarities may be included in the electrode group. For example, in some examplary embodiments, each electrode group may include two electrodes, respectively, and when the electrode group receives radio frequency energy, the two electrodes corresponding to the electrode group may correspond to the opposite polarities respectively. For example, as shown in Fig. 3, the point electrode 122a and the point electrode 122b may be located in the same electrode group, and the point electrode 122c and the point electrode 122d may be located in the same electrode group; when radio frequency energy is received, the point electrode 122a and the point electrode 122b can correspond to opposite polarities respectively; the point electrode 122c and the point electrode 122d may correspond to opposite polarities, respectively. In this case, the electrode group is capable of coupling radio frequency energy to the skin tissue in contact with the electrode group and generating a radio frequency current.

In some examplary embodiments, a plurality of electrodes of at least one electrode group may be in good contact with the skin during the use of the temperature-controlled radio frequency cosmetic device 1. Therefore, the temperature-controlled radio frequency cosmetic device 1 can be used more safely.

In the example according to the embodiment, the temperature-controlled radio frequency cosmetic device 1 may further include a detection module 117 (see Fig. 5) for detecting whether the stimulation head 122 is in good contact with the skin or not.

In some examplary embodiments, the detection module 117 may be a current detecting device connected to each electrode and capable of detecting the magnitude of a current flowing through each electrode. For example, in some examplary embodiments, when the stimulation head 122 receives radio frequency energy and couples the radio frequency energy to the human body, the detection device may detect the magnitude of the current flowing through each electrode in real-time. In some examplary embodiments, the detection device may compare the magnitude of the current flowing through each electrode with a preset range of values respectively, and if the current magnitude of an electrode is not within the preset range of values, the detection device may enable the radio frequency generation module 112 to stop outputting radio frequency energy to the electrode. For example, if the current magnitude of an electrode is not within a preset range of values, the detection device may send a feedback signal to the radio frequency generation module 112, and the radio frequency generation module 112 may receive the feedback signal and turn off the radio frequency output point corresponding to the electrode to stop outputting radio frequency energy.

In some examplary embodiments, the preset range of values may be set by relevant technical personnel, for example, based on the skin impedance of the user.

In other examplary embodiments, the detection module 117 may also be a pressure device that can sense pressure. In some examplary embodiments, the pressure device may be connected to each electrode. For example, the pressure device may be disposed at the rear end of each electrode in the stimulation head 122. In some examplary embodiments, the stimulation head 122 may be subjected to skin pressure against the stimulation head 122 after contacting the skin. The pressure device can control whether each electrode in the stimulation head 122 can receive radio frequency energy or not according to the magnitude of the pressure. For example, the pressure device may control whether the electrode is in communication with a corresponding radio frequency output point or not based on the magnitude of the pressure.

In some examplary embodiments, if the detection module 117 detects that an electrode is not in good contact with the skin, the radio frequency generation module 112 may stop outputting radio frequency energy to the electrode group to which the electrode belongs. For example, the radio frequency generation module 112 may turn off the radio frequency output point corresponding to the electrode to stop outputting radio frequency energy.

In some examplary embodiments, the temperature-controlled radio frequency cosmetic device 1 may also include a temperature sensing module 123 (see Fig. 5) for measuring skin surface temperature in real-time. In some examplary embodiments, the temperature sensing module 123 may be configured at the head 120. In some examplary embodiments, the temperature sensing module 123 may be connected to the control module 113 (see Fig. 5, described hereafter).

In some examplary embodiments, the temperature sensing module 123 may be contact measurement equipment. For example, the temperature sensing module 123 may be a contact temperature sensor (e.g., a thermistor, etc.). Therefore, a more accurate measured temperature can be obtained. That is, when the temperature-controlled radio frequency cosmetic device 1 applies radio frequency energy to the skin to be treated, the probe end 1231 in the temperature sensing module 123 can simultaneously contact the skin surface to measure the skin temperature.

In some examplary embodiments, the temperature sensing module 123 may have a probe end 1231 for contacting skin surface to measure the skin temperature. In some examplary embodiments, the probe end 1231 may be disposed to protrude upwardly from the upper surface 1212a of the functional end 1212. In some examplary embodiments, the probe end 1231 may be disposed at any location on the upper surface 1212a of the functional end 1212 suitable for detecting skin surface temperature. For example, in some examplary embodiments, the probe end 1231 may be located at the center of the upper surface 1212a of functional end 1212. In some examplary embodiments, the height at which the probe end 1231 protrudes relative to the upper surface 1212a may be no greater than the height at which the stimulation head 122 protrudes relative to the upper surface 1212a. In some examplary embodiments, the height at which the probe end 1231 protrudes relative to the upper surface 1212a may be no less than 1/4 of the height at which the stimulation head 122 protrudes relative to the upper surface 1212a. In this case, when the temperature-controlled radio frequency cosmetic device 1 is used, it can be ensured that the stimulation head 122 and the probe end 1231 simultaneously contact the skin surface to be treated.

Fig. 4 is a view showing the structural block diagram of a temperature-controlled radio frequency cosmetic device 1 with a cover 130 according to an embodiment of the present disclosure.

In some examplary embodiments, the temperature-controlled radio frequency cosmetic device 1 further includes a cover 130 (see Fig. 4) for shielding the stimulation head 122 and the probe end 1231. In some examplary embodiments, the cover 130 may be substantially columnar. In some examplary embodiments, one end of the cover 130 may have a groove that matches the periphery of the functional end 1212. In this case, the cover 130 is detachably fixed to the head 120 by means of engagement. In some examplary embodiments, the end face of one end of the cover 130 remote from the head 120 may be horizontal. In this case, when the cover 130 is fixed to the head 120, the end face of one end remote from the head 120 may serve as a supporting face so that the temperature-controlled radio frequency cosmetic device 1 can be placed on horizontal table face in a vertical manner.

Fig. 5 is a view showing the structural block diagram of a temperature-controlled radio frequency cosmetic device 1 according to an embodiment of the present disclosure.

In some examplary embodiments, the temperature-controlled radio frequency cosmetic device 1 may also have a radio frequency generation module 112 (see Fig. 5). Wherein, the radio frequency generation module 112 may be connected to the stimulation head 122 for providing radio frequency energy to the stimulation head 122. In some examplary embodiments, the radio frequency generation module 112 may be configured within the main body portion 110. In some examplary embodiments, the radio frequency generation module 112 may generate radio frequency energy having a particular frequency. In some examplary embodiments, the radio frequency generation module 112 is adapted to output in the 0.5-5MHZ frequency range. For example, the radio frequency generation module 112 may generate a sinusoidal voltage of 1MHz as radio frequency energy. In this case, the stimulation head 122 can receive radio frequency energy having a suitable frequency and can heat the contacted skin surface more effectively. Radio frequency current can be formed once radio frequency energy is applied to the skin surface, and the radio frequency current can heat the dermis layer through the skin surface.

In some examplary embodiments, the radio frequency generation module 112 may be connected to a power module 115 such that the power module 115 supplies energy to the radio frequency generation module 112.

In some examplary embodiments, the temperature-controlled radio frequency cosmetic device 1 may also have a control module 113 (see Fig. 5). In some examplary embodiments, the control module 113 may be configured within the main body portion 110.

In some examplary embodiments, the control module 113 may be connected to the radio frequency generation module 112. In some examplary embodiments, the control module 113 may be used to adjust the duty cycle of the radio frequency generation module 112. The duty cycle may be the output time of the radio frequency generation module 112 in unit time. In some examplary embodiments, the unit time may be determined by a preset frequency (described hereafter).

In some examplary embodiments, the control module 113 may be connected to the temperature sensing module 123. In some examplary embodiments, the temperature sensing module 123 may sense the temperature of the skin surface being treated in real-time. In some examplary embodiments, the control module 113 may sample the real-time temperature sensed by the temperature sensing module 123 at a preset frequency to obtain temperature information. In some examplary embodiments, the preset frequency may be 3-6 times/S. For example, the control module 113 may sample the real-time temperature sensed by the temperature sensing module 123 at a frequency of 4 times/S to obtain 4 groups of temperature information, that is, the control module 113 may collect from the temperature sensing module 123 every 250ms to obtain one group of temperature information. In some examplary embodiments, each group of temperature information may contain a plurality of temperature readings. For example, each group of temperature information may contain 5 temperature readings. In some examplary embodiments, the temperature readings in each group of temperature information may be continuous on the time axis.

In some examplary embodiments, the control module 113 may process each group of temperature information to obtain corresponding target temperature information. For example, in some examplary embodiments, the control module 113 may discard the maximum and minimum values from each group of temperature information, respectively. In this case, the subsequent adjustment of the radio frequency generation module 112 can be facilitated more effectively.

In some examplary embodiments, the control module 113 may adjust the output time (i.e., duty cycle) of the radio frequency generation module 112 in unit time based on the collected temperature information or target temperature information and a temperature threshold. In some examplary embodiments, the unit time may be a collecting cycle of the control module 113 collecting temperature information, i.e., may be determined by a preset frequency. For example, if the preset frequency is 4 times/S, the collecting cycle is 250ms, that is, the unit time is 250ms.

In some examplary embodiments, the control module 113, after collecting the temperature information, may adjust the output time of the radio frequency generation module 112 in an adjacent collecting cycle of a collecting time corresponding to the temperature information according to the temperature information. That is, after the control module 113 collects the temperature information, the control module 113 can adjust the output time of the radio frequency generation module 112 in the subsequent adjacent collecting cycle of the collecting time.

In some examplary embodiments, the control module 113 may average a plurality of temperature readings in each group of target temperature information to obtain a target temperature value corresponding to each group of target temperature information. In some examplary embodiments, the control module 113 may compare the target temperature value with a temperature threshold to adjust the output time of the radio frequency generation module 112 in a subsequent adjacent collecting cycle of the collecting time of the target temperature value. For example, in some examplary embodiments, when the target temperature value is greater than the temperature threshold, the control module 113 may reduce the output time of the radio frequency generation module 112 in the subsequent adjacent collecting cycle; when the target temperature value is less than the temperature threshold, the control module 113 may increase the output time of the radio frequency generation module 112 in the subsequent adjacent collecting cycle.

In other examplary embodiments, the control module 113 may compare a plurality of temperature readings in each group of target temperature information with a temperature threshold respectively to obtain a ratio that the temperature data in the group of target temperature information is greater than the temperature threshold, and the control module 113 may adjust the output time of the radio frequency generation module 112 in a subsequent group of collecting cycle corresponding to the group of target temperature information according to the ratio.

In some examplary embodiments, the temperature threshold may be determined based on an upper limit of the acceptable temperature for the skin tissue. In some examplary embodiments, the temperature threshold may be 40~43 °C. For example, the temperature threshold may be 40°C, 40.2°C, 40.4°C, 40.6°C, 40.8°C, 41°C, 41.2°C, 41.4°C, 41.6°C, 41.8°C, 42°C, 42.2°C, 42.4°C, 42.6°C, 42.8°C or 43°C, etc.

In some examplary embodiments, the temperature-controlled radio frequency cosmetic device 1 may also have an adjustment element 114 (see Figs. 2 and 5) connected to the control module 113. In some examplary embodiments, adjustment element 114 may be a mechanical switch having mechanical contacts. Alternatively, the adjustment element 114 may also be an electronic switch that detects finger contact and switches electronically. In some examplary embodiments, the adjustment element 114 may be disposed on the first housing 111. For example, referring to Fig. 2, a window for assembling the adjustment element 114 may be disposed on the end face of the distal end side 111b of the first housing 111. The adjustment element 114 may be a key switch. The adjustment element 114 may be assembled at the window in a vertically pressed (direction Z) manner.

In some examplary embodiments, the control module 113 may control and adjust the highest output state of the radio frequency generation module 112 through adjusting the adjustment element 114. Specifically, adjusting the adjustment element 114 through a preset adjustment mode may enable the control module 113 to control and adjust the radio frequency generation module 112 to a corresponding highest output state. Therefore, the highest output state of the radio frequency generation module 112 can be adjusted by the adjustment element 114. For example, in some examplary embodiments, the radio frequency generation module 112 may be in a non-output state (i.e., an off state) before the temperature-controlled radio frequency cosmetic device 1 is started. An user can put the temperature-controlled radio frequency cosmetic device 1 into an operating mode via pressing the adjustment element 114 for a long time, i.e. the radio frequency generation module 112 is in an output-enable state (i.e. an on state). In some examplary embodiments, a plurality of different levels may be set in the operating mode. Different levels may limit the highest output state of the radio frequency generation module 112. That is, different ;evels may limit the maximum output time within unit time when the radio frequency generation module 112 is in that level. For example, in some examplary embodiments, the plurality of different levels may be a high level, a medium level, and a low level, respectively. Among them, the high level means that the maximum output time in unit time is allowed to be long when the radio frequency generation module 112 is in this level. Compared with the other levels, the maximum output time allowed by the high level is the longest, the medium level the second, and the low level the shortest. In some examplary embodiments, after the radio frequency generation module 112 is started, the user may configure the temperature-controlled radio frequency cosmetic device 1 in a different level by shortly pressing the adjustment element 114. In some examplary embodiments, after the radio frequency generation module 112 is started, the user may also configure the radio frequency generation module 112 in an off state by long-pressing the adjustment element 114.

In some examplary embodiments, the radio frequency generation module 112 may operate at the maximum output time allowed by the corresponding level before the skin surface temperature reaches the temperature threshold.

In some examplary embodiments, if a corresponding level is adjusted by the adjustment element 114, the control module 113 may subsequently adjust the output time of the radio frequency generation module 112 in unit time not exceed the maximum output time corresponding to the level.

In some examplary embodiments, the control module 113 may be connected to the power module 115 (described in more detail later) to enable the power module 115 to supply energy.

In some examplary embodiments, the temperature-controlled radio frequency cosmetic device 1 may also have a timing module 116 (see Fig. 5) connected to the control module 113. In some examplary embodiments, the timing module 116 may be disposed in the main body housing. For example, the timing module 116 may be disposed in the main body portion 110. In some examplary embodiments, when the radio frequency generation module 112 is in an output state by adjusting the adjustment element 114, the timing module 116 may begin to time until the radio frequency generation module 112 is in an off state. In some examplary embodiments, the control module 113 may control the radio frequency generation module 112 to stop outputting if the timing module 116 times for more than a first preset time. Specifically, if the timing module 116 times for more than a first preset time, the timing module 116 may send a feedback signal to the control module 113, which may receive the feedback signal and control the radio frequency generation module 112 to stop outputting so that the radio frequency generation module 112 is in an off state. In this case, the continuous use time of the temperature-controlled radio frequency cosmetic device 1 can be limited, so that the temperature-controlled radio frequency cosmetic device 1 can be used more safely.

In some examplary embodiments, the temperature-controlled radio frequency cosmetic device 1 may also have a first display module for reflecting level information and a second display module for reflecting the operating state of the radio frequency generation module 112.

In some examplary embodiments, the first display module may be connected to the adjustment element 114 and/or the control module 113 to obtain and display the level information. For example, in some examplary embodiments, the first display module may be a first display light (see Fig. 2) reflecting level information at a frequency at which the first display light blinks. Specifically, if the corresponding level is higher, the frequency of a blinking of the first display lamp is faster. In some examplary embodiments, the first display module may also reflect whether the temperature-controlled radio frequency cosmetic device 1 is in an on state or not.

In other examplary embodiments, the first display module may also be other display devices, for example, the first display module may be a display panel on which the level information is directly displayed with digits or characters.

In some examplary embodiments, the first display module may be disposed on the first housing 111. For example, referring to Fig. 2, the first display module may be disposed on the periphery of the first housing 111.

In some examplary embodiments, the second display module may be connected to the adjustment element 114 and/or the control module 113 to obtain the operating state of the radio frequency generation module 112. For example, the second display module can be a second display light, and if the radio frequency generation module 112 is in an off state, the second display light is in an off state; if the radio frequency generation module 112 is in an operating state, the second display light is in a bright state. In some examplary embodiments, the first display light and the second display light may display different colors.

In other examplary embodiments, the second display module may also be other display devices, for example, the second display module may be a display panel on which the operating state of the radio frequency generation module 112 is directly displayed with digits or characters.

In some examplary embodiments, the second display module may be disposed on the first housing 111. For example, referring to Fig. 2, the second display module may be disposed on the periphery of the first housing 111.

In some examplary embodiments, the power module 115 may be a power interface connected to an external power supply to enable the external power supply to supply energy to the temperature-controlled radio frequency cosmetic device 1 (see FIGS. 2 and 5). Therefore, the external power supply can be enabled to directly supply energy to the temperature-controlled radio frequency cosmetic device 1. In some examplary embodiments, the power interface may be disposed at the main body portion 110. For example, referring to Fig. 2, the power interface may be disposed on the end face of the distal end side 111b of the main body portion 110.

In other examplary embodiments, the power module 115 may also be energy supply equipment such as batteries. In some examplary embodiments, the power module 115 may be disposed in the temperature-controlled radio frequency cosmetic device 1. For example, the power module 115 may be disposed in the main body portion 110. In some examplary embodiments, the power module 115 may be rechargeable energy supply equipment. For example, the power module 115 may be a rechargeable lithium battery. In this case, the temperature-controlled radio frequency cosmetic device 1 may also have a charging port for connecting an external power supply and being capable of charging the power module 115.

Hereinafter, a method of using the temperature-controlled radio frequency cosmetic device 1 according to an example of the present embodiment will be described in detail with reference to the accompanying drawings.

In some examplary embodiments, the user may handhold the temperature-controlled radio frequency cosmetic device 1 and may treat the skin by adjusting the temperature-controlled radio frequency cosmetic device 1.

In some examplary embodiments, the user may place the temperature-controlled radio frequency cosmetic device 1 in the operating state by adjusting a switch so that the stimulation head 122 contacts the skin to be treated and remains continuously moving. In some examplary embodiments, the user may apply a skin treating product such as a gel, etc. to the skin to be treated prior to using the temperature-controlled radio frequency cosmetic device 1. Therefore, the skin can be protected more effectively.

Specifically, the user can firstly apply a skin treating product such as gel, etc. to the skin to be treated, then plug in the power supply and turn on the temperature-controlled radio frequency cosmetic device 1 by long-pressing the adjustment element 114, then the user can select an appropriate level by shortly pressing the adjustment element 114 to treat the skin according to the sensitive condition of the skin, and after selecting a level, the user can bring the stimulation head 122 and the probe end 1231 of the temperature sensing module 123 into contact with the skin (e.g., the front end of the stimulation head 122 may be placed vertically on the skin) and keep a spiral movement to make the stimulation head 122 move on the skin surface. The user with more sensitive skin can select a lower level.

In some examplary embodiments, the stimulation head 122 may be kept in good contact with the skin while the probe end 1231 of the temperature sensing module 123 may be kept in contact with the skin during the spiral movement of the temperature-controlled radio frequency cosmetic device 1 by the user.

In some examplary embodiments, the time that the user can continue the spiral movement in the same skin region may be a second preset time. For example, the user may continue the spiral movement for 3~6min in the same skin region. In some examplary embodiments, the temperature-controlled radio frequency cosmetic device 1 may be automatically turned off if its on-time exceeds a first preset time. In some examplary embodiments, if the use of the temperature-controlled radio frequency cosmetic device 1 is complete, the user may stop the operation of the temperature-controlled radio frequency cosmetic device 1 by long-pressing the adjustment element 114.

The invention is defined by claim 1. Preferred embodiments are defined in the dependent claims. Further aspects, examples and embodiments disclosed herein are for exemplary purpose only and do not form part of the invention.

## Claims

1. A temperature-controlled radio frequency cosmetic device (1) capable of monitoring a surface temperature of a treated skin in real-time and keeping at appropriate temperature, comprising
a main body portion (110) used for hand-holding, a head portion (120) connected with the main body portion (110) having a functional end (1212), a stimulation head (122) which is in contact with skin surface and couples radio frequency energy to a human body, a radio frequency generation module (112) which provides radio frequency energy for the stimulation head (122), a control module (113) which is connected with the radio frequency generation module (112) and is used for controlling an output state of the radio frequency generation module (112), a power module (115) which is used for supplying energy, and a temperature sensing module (123) having a probe end (1231) for contacting the skin surface which is in contact with the skin surface and measures skin surface temperature in real-time;
wherein,
the stimulation head (122) is disposed on an upper surface (1212a) of the functional end (1212), the probe end (1231) of the temperature sensing module (123) is located at the center of the upper surface (1212a) of the functional end (1212), the stimulation head (122) comprises a plurality of electrode groups,
when the temperature-controlled radio frequency cosmetic device (1) operates,
the stimulation head (122) and the probe end (1231) of the temperature sensing module (123) simultaneously contact the skin surface,
at least one electrode group in the stimulation head (122) contacts the skin surface and receives radio frequency energy from the radio frequency generation module (112) to generate radio frequency current for stimulating skin tissue,
and the temperature sensing module (123) measures the surface temperature of the skin tissue in real-time,
and the control module (113) is connected with the temperature sensing module (123) and samples the real-time temperature measured by the temperature sensing module (123) at a preset frequency to collect a plurality of groups of temperature information including a plurality of temperatures respectively,
and the control module (113) processes each group of temperature information to collect a plurality of corresponding desired group of temperature information,
and the control module (113) averages the plurality of temperatures of each desired group of temperature information to collect a corresponding desired temperature value,
and the control module (113) adjusts output time of the radio frequency generation module (112) in a corresponding subsequent collection period after the desired temperature value according to a comparison of the desired temperature value and a temperature threshold,
and the collection period is determined by the preset frequency, and the temperature threshold is determined based on an upper limit of acceptable temperature of the skin tissue,
when the desired temperature value is greater than the temperature threshold, the control module (113) reduces the output time of the radio frequency generation module (112) in the corresponding subsequent collection period after the desired temperature value, and
when the desired temperature value is less than the temperature threshold, the control module (113) increases the output time of the radio frequency generation module (112) in the corresponding subsequent collection period after the desired temperature value.

2. The temperature-controlled radio frequency cosmetic device according to claim 1, wherein
the device (1) further comprises a detection module (117) for detecting whether the stimulation head (122) is in good contact with the skin surface or not,
wherein the detection module (117) is a current detection device which is connected with electrodes in each electrode group and configured to detect a magnitude of a current flowing through each electrode,
and when the stimulation head (122) receives the radio frequency energy and couples the radio frequency energy to a human body,
the detection device detects the magnitude of the current flowing through each electrode in real-time,
and the detection module (117) compares the magnitude of the current flowing through each electrode with a preset value range respectively,
and if the magnitude of the current of an electrode is not in a preset value range, the detection device enables the radio frequency generation module (112) to stop outputting the radio frequency energy to the electrode.

3. The temperature-controlled radio frequency cosmetic device according to claim 1, wherein
the device (1) further comprises a detection module (117) for detecting whether the stimulation head is in good contact with the skin surface or not,
wherein the detection module (117) is a pressure device configured to sense pressure and is connected with each electrode,
when the stimulation head (122) is in contact with the skin surface, the stimulation head (122) is subjected to skin pressure against the stimulation head (122),
and the pressure device controls whether an electrode receives radio frequency energy according to the magnitude of pressure applied to each electrode in the stimulation head (122) or not.

4. The temperature-controlled radio frequency cosmetic device according to claim 1, wherein
the device (1) further comprises an adjustment element (114) connected with the control module (113), wherein by adjusting the adjustment element (114), the control module (113) is enabled to control and adjust a highest output state of the radio frequency generation module (112).

5. The temperature-controlled radio frequency cosmetic device according to claim 4, wherein
the device (1) further comprises a timing module (116) connected with the control module (113),
wherein when the radio frequency generation module (112) is in an on state by adjusting the adjustment element (114), the timing module (116) starts timing until the radio frequency generation module (112) is in an off state,
and if the recorded time of the timing module (116) exceeds a first preset time, the timing module (116) sends feedback signal to the control module (113),
the control module (113) receives the feedback signal and controls the radio frequency generation module (112) to stop outputting so that the radio frequency generation module (112) is in an off state.

6. The temperature-controlled radio frequency cosmetic device according to claim 1, wherein
the power module (115) is a power interface connected with an external power supply.

7. The temperature-controlled radio frequency cosmetic device according to claim 1, wherein
the temperature sensing module (123) is a contact temperature sensor.

8. The temperature-controlled radio frequency cosmetic device according to claim 1, wherein
the radio frequency generation module (112) is applicable to output in a 0.5~5MHZ frequency range.

9. The temperature-controlled radio frequency cosmetic device according to claim 1, wherein
the radio frequency generation module (112) comprises a plurality of radio frequency output points, and each radio frequency output point corresponds to a plurality of electrodes in the stimulation head (122).

10. The temperature-controlled radio frequency cosmetic device according to claim 9, wherein
a polarity of the point electrodes sharing the same radio frequency output point are the same.

11. The temperature-controlled radio frequency cosmetic device according to claim 10, wherein
when an electrode group receives radio frequency energy from the radio frequency generation module (112), at least two electrodes of opposite polarities are included in the electrode group.

12. The temperature-controlled radio frequency cosmetic device according to claim 1, wherein
the temperature threshold is 40 - 43 degrees, the preset frequency is 4 times per second, and the collection period is 250 milliseconds.

13. The temperature-controlled radio frequency cosmetic device according to claim 1, wherein
the control module (113) discards the maximum and minimum values from each group of temperature information respectively to obtain corresponding desired temperature information.

14. The temperature-controlled radio frequency cosmetic device according to claim 4, wherein
the temperature-controlled radio frequency cosmetic device (1) is set in different levels through adjusting the adjustment element (114), and the temperature-controlled radio frequency cosmetic device (1) has a first display module reflecting level information.

## Patentansprüche

1. Temperaturgesteuerte Funkfrequenzkosmetikvorrichtung (1), die in der Lage ist, eine Oberflächentemperatur einer behandelten Haut in Echtzeit zu überwachen und auf einer geeigneten Temperatur zu halten, die Folgendes umfasst
einen Hauptkörperabschnitt (110), der zum Halten mit der Hand verwendet wird, einen Kopfabschnitt (120), der mit dem Hauptkörperabschnitt (110) verbunden ist und ein Funktionsende (1212) aufweist, einen Stimulationskopf (122), der mit einer Hautoberfläche verbunden ist und Funkfrequenzenergie an einen menschlichen Körper koppelt, ein Funkfrequenzerzeugungsmodul (112), das Funkfrequenzenergie für den Stimulationskopf (122) bereitstellt, ein Steuermodul (113), das mit dem Funkfrequenzerzeugungsmodul (112) verbunden ist und zum Steuern eines Ausgabezustands des Funkfrequenzerzeugungsmoduls (112) verwendet wird, ein Leistungsmodul (115), das zum Zuführen von Energie verwendet wird, und ein Temperaturerfassungsmodul (123) mit einem Sondenende (1231) zum Kontaktieren der Hautoberfläche, das mit der Hautoberfläche in Kontakt ist und eine Hautoberflächentemperatur in Echtzeit misst;
wobei,
der Stimulationskopf (122) auf einer oberen Oberfläche (1212a) des Funktionsendes (1212) angeordnet ist, das Sondenende (1231) des Temperaturerfassungsmoduls (123) sich in der Mitte der oberen Oberfläche (1212a) des Funktionsendes (1212) befindet, der Stimulationskopf (122) eine Vielzahl von Elektrodengruppen umfasst,
wenn die temperaturgesteuerte Funkfrequenzkosmetikvorrichtung (1) betrieben wird,
der Stimulationskopf (122) und das Sondenende (1231) des Temperaturerfassungsmoduls (123) die Hautoberfläche gleichzeitig kontaktieren,
mindestens eine Elektrodengruppe im Stimulationskopf (122) die Hautoberfläche kontaktiert und Funkfrequenzenergie vom Funkfrequenzerzeugungsmodul (112) empfängt, um einen Funkfrequenzstrom zum Stimulieren eines Hautgewebes zu erzeugen,
und das Temperaturerfassungsmodul (123) die Oberflächentemperatur des Hautgewebes in Echtzeit misst,
und das Steuermodul (113) mit dem Temperaturerfassungsmodul (123) verbunden ist und die Echtzeittemperatur, die vom Temperaturerfassungsmodul (123) gemessen wird, mit einer voreingestellten Frequenz abtastet, um eine Vielzahl von Gruppen von Temperaturinformationen, die jeweils eine Vielzahl von Temperaturen beinhalten, zu sammeln,
und das Steuermodul (113) jede Gruppe von Temperaturinformationen verarbeitet, um eine Vielzahl von entsprechenden gewünschten Gruppen von Temperaturinformationen zu sammeln,
und das Steuermodul (113) die Vielzahl von Temperaturen jeder gewünschten Gruppe von Temperaturinformationen mittelt, um einen entsprechenden gewünschten Temperaturwert zu sammeln,
und das Steuermodul (113) eine Ausgabezeit des Funkfrequenzerzeugungsmoduls (112) in einer entsprechenden nachfolgenden Sammelperiode nach dem gewünschten Temperaturwert gemäß einem Vergleich des gewünschten Temperaturwertes und eines Temperaturschwellwerts anpasst,
und die Sammelperiode durch die voreingestellte Frequenz bestimmt wird und der Temperaturschwellwert auf Basis einer oberen Grenze einer akzeptablen Temperatur des Hautgewebes bestimmt wird,
wenn der gewünschte Temperaturwert größer ist als der Temperaturschwellwert, das Steuermodul (113) die Ausgabezeit des Funkfrequenzerzeugungsmoduls (112) in der entsprechenden nachfolgenden Sammelperiode nach dem gewünschten Temperaturwert reduziert, und
wenn der gewünschte Temperaturwert kleiner ist als der Temperaturschwellwert, das Steuermodul (113) die Ausgabezeit des Funkfrequenzerzeugungsmoduls (112) in der entsprechenden nachfolgenden Sammelperiode nach dem gewünschten Temperaturwert erhöht.

2. Temperaturgesteuerte Funkfrequenzkosmetikvorrichtung nach Anspruch 1, wobei
die Vorrichtung (1) zum Detektieren, ob der Stimulationskopf (122) gut mit der Hautoberfläche in Kontakt ist oder nicht, ferner ein Detektionsmodul (117) umfasst,
wobei das Detektionsmodul (117) eine Stromdetektionsvorrichtung ist, die mit Elektroden in jeder Elektrodengruppe verbunden und dazu ausgelegt ist, eine Größe eines Stroms, der durch jede Elektrode fließt, zu bestimmen,
und wenn der Stimulationskopf (122) die Funkfrequenzenergie empfängt und die Funkfrequenzenergie an einen menschlichen Körper koppelt,
die Detektionsvorrichtung die Größe des Stroms, der durch jede Elektrode fließt, in Echtzeit misst,
und das Detektionsmodul (117) die Größe des Stroms, der durch jede Elektrode fließt, jeweils mit einem voreingestellten Wertebereich vergleicht,
und wenn die Größe des Stroms einer Elektrode nicht innerhalb eines voreingestellten Wertebereichs liegt, die Detektionsvorrichtung es dem Funkfrequenzerzeugungsmodul (112) ermöglicht, das Ausgeben der Funkfrequenzenergie an die Elektrode zu stoppen.

3. Temperaturgesteuerte Funkfrequenzkosmetikvorrichtung nach Anspruch 1, wobei
die Vorrichtung (1) zum Detektieren, ob der Stimulationskopf gut mit der Hautoberfläche in Kontakt ist oder nicht, ferner ein Detektionsmodul (117) umfasst,
wobei das Detektionsmodul (117) eine Druckvorrichtung ist, die dazu ausgelegt ist, Druck zu erfassen, und mit jeder Elektrode verbunden ist,
wenn der Stimulationskopf (122) mit der Hautoberfläche in Kontakt ist, der Stimulationskopf (122) einem Hautdruck gegen den Stimulationskopf (122) unterliegt,
und die Druckvorrichtung gemäß der Größe eines Drucks, der auf jede Elektrode im Stimulationskopf (122) ausgeübt wird, steuert, ob eine Elektrode Funkfrequenzenergie empfängt oder nicht.

4. Temperaturgesteuerte Funkfrequenzkosmetikvorrichtung nach Anspruch 1, wobei
die Vorrichtung (1) ferner ein Anpassungselement (114) umfasst, das mit dem Steuermodul (113) verbunden ist, wobei es durch Anpassen des Anpassungselements (114) dem Steuermodul (113) möglich ist, einen höchsten Ausgabezustand des Funkfrequenzerzeugungsmoduls (112) zu steuern und anzupassen.

5. Temperaturgesteuerte Funkfrequenzkosmetikvorrichtung nach Anspruch 4, wobei
die Vorrichtung (1) ferner ein Zeitsteuerungsmodul (116) umfasst, das mit dem Steuermodul (113) verbunden ist,
wobei, wenn sich das Funkfrequenzerzeugungsmodul (112) aufgrund des Anpassens des Anpassungselements (114) in einem Einzustand befindet, das Zeitsteuerungsmodul (116) eine Zeitsteuerung startet, bis sich das Funkfrequenzerzeugungsmodul (112) in einem Auszustand befindet,
und wenn die aufgezeichnete Zeit des Zeitsteuerungsmoduls (116) eine erste voreingestellte Zeit überschreitet, sendet das Zeitsteuerungsmodul (116) ein Rückmeldesignal an das Steuermodul (113),
das Steuermodul (113) empfängt das Rückmeldesignal und steuert das Funkfrequenzerzeugungsmodul (112), um das Ausgeben zu stoppen, derart, dass sich das Funkfrequenzerzeugungsmodul (112) in einem Auszustand befindet.

6. Temperaturgesteuerte Funkfrequenzkosmetikvorrichtung nach Anspruch 1, wobei
das Leistungsmodul (115) eine Leistungsschnittstelle ist, die mit einer externen Leistungsversorgung verbunden ist.

7. Temperaturgesteuerte Funkfrequenzkosmetikvorrichtung nach Anspruch 1, wobei
das Temperaturerfassungsmodul (123) ein Kontakttemperatursensor ist.

8. Temperaturgesteuerte Funkfrequenzkosmetikvorrichtung nach Anspruch 1, wobei
das Funkfrequenzerzeugungsmodul (112) anwendbar ist, um in einem Frequenzbereich von 0,5~5 MHz auszugeben.

9. Temperaturgesteuerte Funkfrequenzkosmetikvorrichtung nach Anspruch 1, wobei
das Funkfrequenzerzeugungsmodul (112) eine Vielzahl von Funkfrequenzausgabepunkten umfasst und jeder Funkfrequenzausgabepunkt einer Vielzahl von Elektroden im Stimulationskopf (122) entspricht.

10. Temperaturgesteuerte Funkfrequenzkosmetikvorrichtung nach Anspruch 9, wobei
eine Polarität der Punktelektroden, die denselben Funkfrequenzausgabepunkt gemeinsam verwenden, dieselbe ist.

11. Temperaturgesteuerte Funkfrequenzkosmetikvorrichtung nach Anspruch 10, wobei
wenn eine Elektrodengruppe Funkfrequenzenergie vom Funkfrequenzerzeugungsmodul (112) empfängt, mindestens zwei Elektroden entgegengesetzter Polaritäten in der Elektrodengruppe beinhaltet sind.

12. Temperaturgesteuerte Funkfrequenzkosmetikvorrichtung nach Anspruch 1, wobei
der Temperaturschwellwert 40-43 Grad beträgt, die voreingestellte Frequenz 4 Mal pro Sekunde beträgt und die Sammelperiode 250 Millisekunden beträgt.

13. Temperaturgesteuerte Funkfrequenzkosmetikvorrichtung nach Anspruch 1, wobei
das Steuermodul (113) maximale und minimale Werte jeweils aus jeder Gruppe von Temperaturinformationen verwirft, um entsprechende gewünschte Temperaturinformationen zu erhalten.

14. Temperaturgesteuerte Funkfrequenzkosmetikvorrichtung nach Anspruch 4, wobei
die temperaturgesteuerte Funkfrequenzkosmetikvorrichtung (1) durch Anpassen des Anpassungselements (114) auf unterschiedliche Niveaus eingestellt wird und die temperaturgesteuerte Funkfrequenzkosmetikvorrichtung (1) ein erstes Anzeigemodul aufweist, das Niveauinformationen reflektiert.

## Revendications

1. Dispositif cosmétique de radiofréquence thermorégulée (1) capable de surveiller une température de surface d'une peau traitée en temps réel et de la maintenir à une température appropriée, comprenant
une partie de corps principal (110) utilisée pour la prise en main, une partie de tête (120) reliée à la partie de corps principal (110) ayant une extrémité fonctionnelle (1212), une tête de stimulation (122) qui est en contact avec la surface de la peau et couple l'énergie de radiofréquence à un corps humain, un module de génération de radiofréquence (112) qui fournit une énergie de radiofréquence pour la tête de stimulation (122), un module de commande (113) qui est relié au module de génération de radiofréquence (112) et est utilisé pour commander un état de sortie du module de génération de radiofréquence (112), un module d'alimentation (115) qui est utilisé pour amener de l'énergie, et un module de détection de température (123) ayant une extrémité de sonde (1231) pour entrer en contact avec la surface de la peau qui est en contact avec la surface de la peau et mesure la température de la surface de la peau en temps réel ;
dans lequel,
la tête de stimulation (122) est disposée sur une surface supérieure (1212a) de l'extrémité fonctionnelle (1212), l'extrémité de sonde (1231) du module de détection de température (123) est située au centre de la surface supérieure (1212a) de l'extrémité fonctionnelle (1212), la tête de stimulation (122) comprend une pluralité de groupes d'électrodes,
lorsque le dispositif cosmétique de radiofréquence thermorégulée (1) fonctionne,
la tête de stimulation (122) et l'extrémité de sonde (1231) du module de détection de température (123) sont simultanément en contact avec la surface de la peau,
au moins un groupe d'électrodes dans la tête de stimulation (122) est en contact avec la surface de la peau et reçoit une énergie de radiofréquence du module de génération de radiofréquence (112) pour générer un courant de radiofréquence pour stimuler le tissu de la peau,
et le module de détection de température (123) mesure la température de surface du tissu de la peau en temps réel,
et le module de commande (113) est relié au module de détection de température (123) et échantillonne la température en temps réel mesurée par le module de détection de température (123) à une fréquence prédéfinie pour collecter une pluralité de groupes d'informations de température incluant une pluralité de températures respectivement,
et le module de commande (113) traite chaque groupe d'informations de température pour collecter une pluralité de groupes souhaités correspondants d'informations de température,
et le module de commande (113) calcule la moyenne de la pluralité de températures de chaque groupe souhaité d'informations de température pour collecter une valeur de température souhaitée correspondante,
et le module de commande (113) règle le temps de sortie du module de génération de radiofréquence (112) dans une période de collecte ultérieure correspondante après la valeur de température souhaitée selon une comparaison de la valeur de température souhaitée et d'un seuil de température,
et la période de collecte est déterminée par la fréquence prédéfinie, et le seuil de température est déterminé en fontion d'une limite supérieure de température acceptable du tissu de la peau,
lorsque la valeur de température souhaitée est supérieure au seuil de température, le module de commande (113) réduit le temps de sortie du module de génération de radiofréquence (112) dans la période de collecte ultérieure correspondante après la valeur de température souhaitée, et
lorsque la valeur de température souhaitée est inférieure au seuil de température, le module de commande (113) augmente le temps de sortie du module de génération de radiofréquence (112) dans la période de collecte ultérieure correspondante après la valeur de température souhaitée.

2. Dispositif cosmétique de radiofréquence thermorégulée selon la revendication 1, dans lequel
le dispositif (1) comprend en outre un module de détection (117) pour détecter si la tête de stimulation (122) est en bon contact avec la surface de la peau ou non,
dans lequel le module de détection (117) est un dispositif de détection de courant qui est relié à des électrodes dans chaque groupe d'électrodes et configuré pour détecter une amplitude d'un courant circulant à travers chaque électrode,
et lorsque la tête de stimulation (122) reçoit l'énergie de radiofréquence et couple l'énergie de radiofréquence à un corps humain,
le dispositif de détection détecte l'amplitude du courant circulant à travers chaque électrode en temps réel,
et le module de détection (117) compare l'amplitude du courant circulant à travers chaque électrode avec une plage de valeurs prédéfinies respectivement,
et si l'amplitude du courant d'une électrode n'est pas dans une plage de valeurs prédéfinie, le dispositif de détection permet au module de génération de radiofréquence (112) d'arrêter la délivrance de l'énergie de radiofréquence à l'électrode.

3. Dispositif cosmétique de radiofréquence thermorégulée selon la revendication 1, dans lequel
le dispositif (1) comprend en outre un module de détection (117) pour détecter si la tête de stimulation est en bon contact avec la surface de la peau ou non,
dans lequel le module de détection (117) est un dispositif de pression configuré pour détecter la pression et est relié à chaque électrode,
lorsque la tête de stimulation (122) est en contact avec la surface de la peau, la tête de stimulation (122) est soumise à une pression de la peau contre la tête de stimulation (122),
et le dispositif de pression commande si une électrode reçoit de l'énergie de radiofréquence selon l'amplitude de pression appliquée à chaque électrode dans la tête de stimulation (122) ou non.

4. Dispositif cosmétique de radiofréquence thermorégulée selon la revendication 1, dans lequel
le dispositif (1) comprend en outre un élément de réglage (114) relié au module de commande (113), dans lequel en réglant l'élément de réglage (114), le module de commande (113) est activé pour commander et régler un état de sortie le plus élevé du module de génération de radiofréquence (112).

5. Dispositif cosmétique de radiofréquence thermorégulée selon la revendication 4, dans lequel
le dispositif (1) comprend en outre un module de temporisation (116) relié au module de commande (113),
dans lequel lorsque le module de génération de radiofréquence (112) est dans un état activé en réglant l'élément de réglage (114), le module de temporisation (116) commence la temporisation jusqu'à ce que le module de génération de radiofréquence (112) soit dans un état désactivé,
et si le temps enregistré du module de temporisation (116) dépasse un premier temps prédéfini, le module de temporisation (116) envoie un signal de rétroaction au module de commande (113),
le module de commande (113) reçoit le signal de rétroaction et commande le module de génération de radiofréquence (112) pour arrêter la délivrance de sorte que le module de génération de radiofréquence (112) est dans un état désactivé.

6. Dispositif cosmétique de radiofréquence thermorégulée selon la revendication 1, dans lequel
le module de puissance (115) est une interface de puissance reliée à une alimentation en énergie externe.

7. Dispositif cosmétique de radiofréquence thermorégulée selon la revendication 1, dans lequel
le module de détection de température (123) est un capteur de température à contact.

8. Dispositif cosmétique de radiofréquence thermorégulée selon la revendication 1, dans lequel
le module de génération de radiofréquence (112) est applicable à une sortie dans une plage de fréquences de 0,5 à environ 5 MHZ.

9. Dispositif cosmétique de radiofréquence thermorégulée selon la revendication 1, dans lequel
le module de génération de radiofréquence (112) comprend une pluralité de points de sortie de radiofréquence, et chaque point de sortie de radiofréquence correspond à une pluralité d'électrodes dans la tête de stimulation (122).

10. Dispositif cosmétique de radiofréquence thermorégulée selon la revendication 9, dans lequel
une polarité des électrodes de point partageant le même point de sortie de radiofréquence sont les mêmes.

11. Dispositif cosmétique de radiofréquence thermorégulée selon la revendication 10, dans lequel
lorsqu'un groupe d'électrodes reçoit une énergie de radiofréquence du module de génération radiofréquence (112), au moins deux électrodes de polarités opposées sont incluses dans le groupe d'électrodes.

12. Dispositif cosmétique de radiofréquence thermorégulée selon la revendication 1, dans lequel
le seuil de température est de 40 à 43 degrés, la fréquence prédéfinie est de 4 fois par seconde et la période de collecte est de 250 millisecondes.

13. Dispositif cosmétique de radiofréquence thermorégulée selon la revendication 1, dans lequel
le module de commande (113) rejette les valeurs maximale et minimale de chaque groupe d'informations de température respectivement pour obtenir des informations de température souhaitées correspondantes.

14. Dispositif cosmétique de radiofréquence thermorégulée selon la revendication 4, dans lequel
le dispositif cosmétique de radiofréquence thermorégulée (1) est fixé à différents niveaux par réglage de l'élément de réglage (114), et le dispositif cosmétique à radiofréquence thermorégulée (1) a un premier module d'affichage reflétant des informations de niveau.
